# EUROPEAN PATENT SPECIFICATION

(11) **EP 2 274 303 B1**
(45) Date of publication and mention of the grant of the patent: **29.08.2012**
(21) Application number: 09728114.1
(22) Date of filing: 31.03.2009
(51) Int. Cl.: C07D 403/06, A61K 31/404, A61P 35/00

(54) **PROCESSES FOR PREPARING SUNITINIB AND SALTS THEREOF**
VERFAHREN ZUR HERSTELLUNG VON SUNITINIB UND SALZEN DAVON
PROCÉDÉS DE PRÉPARATION DE SUNITINIB ET DE SELS DE CE DERNIER

(30) Priority: 17.09.2008 US 97592 P; 10.11.2008 US 113044 P; 22.07.2008 US 82681 P; 21.07.2008 US 82405 P; 04.09.2008 US 94341 P; 14.08.2008 US 88998 P; 31.03.2008 US 41103 P
(43) Date of publication of application: 19.01.2011
(73) Proprietor: Teva Pharmaceutical Industries Ltd., 49131 Petah Tiqva (IL)
(72) Inventor: BIGATTI, Ettore, I-20017 Rho (IT); CANAVESI, Augusto, I-22070 Locate Varesino (CO) (IT); MACDONALD, Peter, Lindsay, CH-6925 Gentilino (CH); SCARPITTA, Francesca, I-10015 Ivrea (TO) (IT)
(74) Representative: Eder, Michael
(86) International application number: PCT/US2009/038934
(87) International publication number: WO 2009/124037

(56) References cited:
- WO-A-2009/067674
- WO-A2-2007/081560
- US-A1- 2003 229 229
- SUN L ET AL: "Discovery of 5-[5-fluoro-2-oxo-1,2- dihydroindol-(3Z)-ylidenemethyl] 2,4- dimethyl-1H-pyrrole-3-carboxylic acid (2-diethylaminoethyl)ami de, a novel tyrosine kinase inhibitor targeting vascular endothelial and platelet-derived growth factor receptor tyrosine kinase" JOURNAL OF MEDICINAL CHEMISTRY, AMERICAN CHEMICAL SOCIETY, WASHINGTON, US, vol. 46, no. 7, 27 March 2003 (2003-03-27), pages 1116-1119, XP009070151 ISSN: 0022-2623

## Description

### CROSS-REFERENCE TO RELATED APPLICATIONS

This application claims the benefit of U.S. Provisional Patent Application Ser. Nos. 61/113,044, filed November 10, 2008; 61/097,592, filed September 17, 2008; 61/094,341, filed September 4, 2008; 61/088,998, filed August 14, 2008; 61/082,681, filed July 22, 2008; 61/082,405, filed July 21, 2008; and 61/041,103, filed March 31, 2008.

### FIELD OF INVENTION

The present invention relates to a process for the preparation of Sunitinib and salt thereof.

### BACKGROUND OF THE INVENTION

Sunitinib base ("Sunitinib") of the following formula: 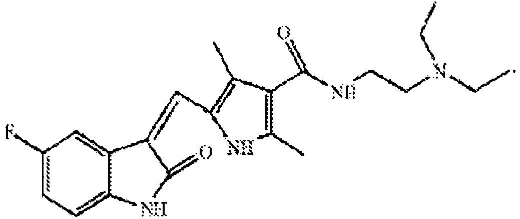 is an intermediate for Sunitinib salts, such as Sunitinib malate of the following formula:

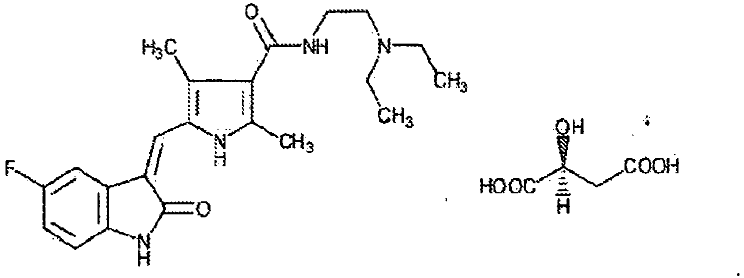

Sunitinib malate is marketed under the trade name Sutent® by Pfizer. It is an oral, multi-targeted tyrosine kinase inhibitor used for treatment of various types of cancer.

Sunitinib and salts thereof, process of preparation thereof and the use of these salts are disclosed in US patent No. 6,573,293 B2 ("US'293").

The preparation of Sunitinib disclosed in US '293 is done by amidation of 5-formyl-2,4-1*H*-pyrrole-3-carboxylic acid to obtain 5-formyl-2, 4-1*H*-pyrrole-3-carboxylic acid (2-diethylaminoethyl) amide in a yield of 43%. The obtained amide is then condensed with 5-fluoro-1, 3-dihydro-indol-2-one in EtOH in the presence of pyrrolidine, obtaining Sunitinib. The process can be illustrated in the following scheme:

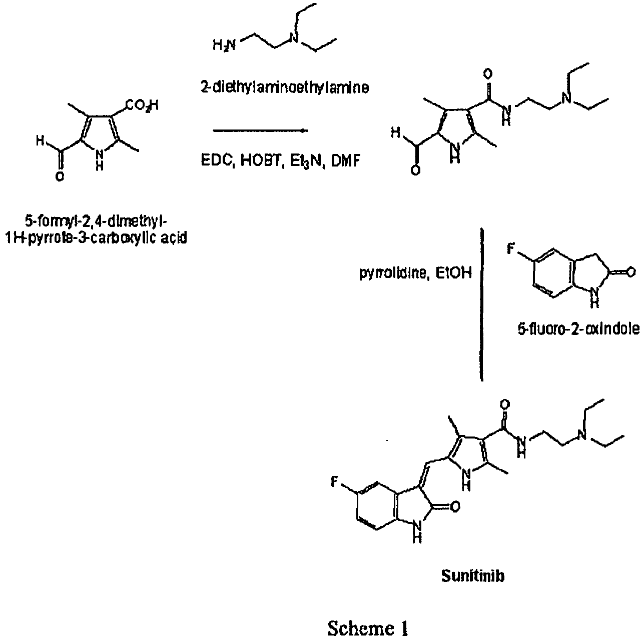

The amidation reaction in US '293 is performed on an activated carboxylic acid derivative. This synthetic approach is also emplyoyed in J. Med.Chem., 2003, 46, 116-1119. According to Journal of Organic Chemistry, 2003, 68, 6447, this reaction leads also to the formation of by-products. In addition, the amide coupling reagents, which are used in US '293 are toxic, dangerous and expensive reagents.

US 2006/0009510 (US '510) and Journal of Organic Chemistry, 2003, 68, 6447 disclose an alternative synthesis for the preparation of Sunitinib by reacting N-[2-(diethylamino) ethyl]-2, 4-dimethyl-1H-pyrrole-3-carboxamide with 5-fluoro-2-oxindole, in a yield of 74%, in the presence of acetonitrile and Vislmeier reagent, as described in the following scheme:

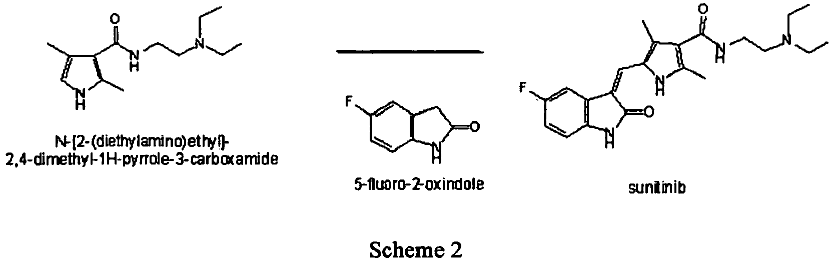

US Patent No. 7,119,209 also discloses an alternative process for the preparation of Sunitinib by first activation of the pyrrole moiety as imidazole derivative, which is then used in the second step for the in situ preparation of the amide, as described in the following scheme:

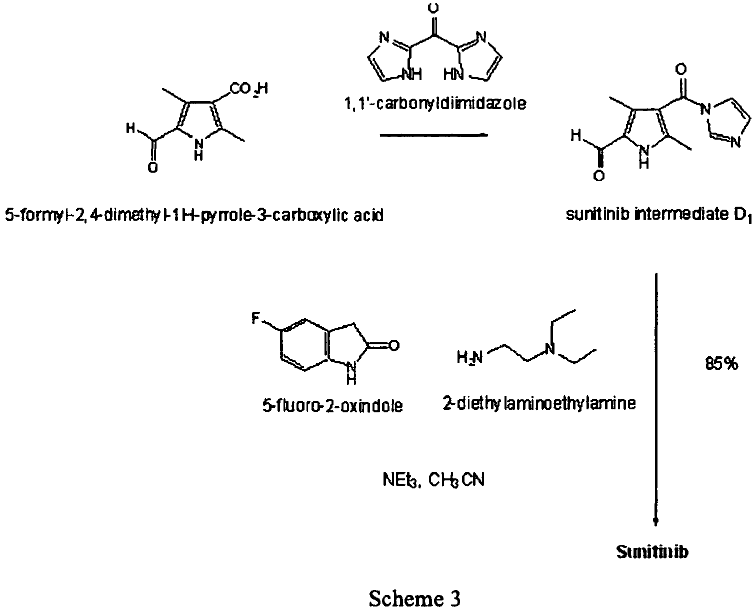

There is a need in the art for an improved process for the preparation of Sunitinib and salts thereof which is also suitable for industrial scale.

### SUMMARY OF THE INVENTION

In one embodiment, the present invention encompasses 5-(5-fluoro-2-oxo-1, 2-dihydro-indol-3Z-ylidenemethyl)-2, 4-dimethyl-1H-pyrrole-3-carbonyl substitute of the following formula 1; 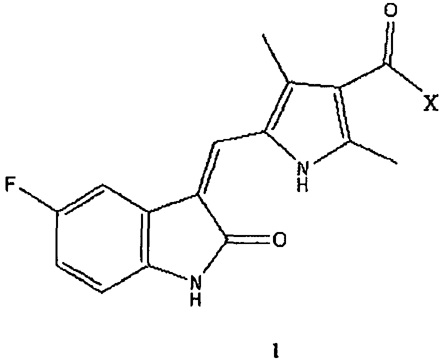 wherein X is either Cl.

In another embodiment, the present invention encompasses the preparation of sunitinib and salts thereof of the following formula: 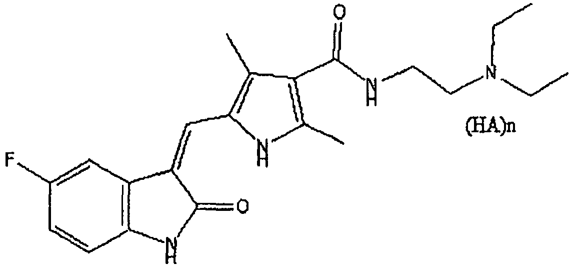 from the compound of formula 1, wherein n is either 0 or 1, HA is a diacid, preferably, malic acid.

In another embodiment, the present invention encompasses a process for preparing 5-(5-fluoro-2-oxo-1,2-dihydro-indol-3Z-ylidenemethyl)-2,4-dimethyl-1H-pyrrole-3-carbonyl substitute of formula 1 comprising reacting 5-(5-fluoro-2-oxo-1,2-dihydro-indol-(3Z)-ylidenemethyl)-2,4-dimethyl-1H-pyrrolo-3-carboxylic acid of formula 4 of the following structure: 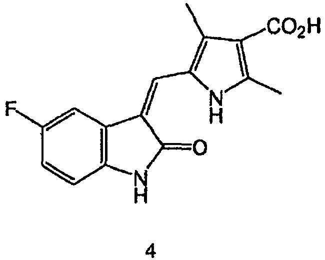 with chlorinating agent

In another embodiment, the present invention encompasses a process for preparing sunitinib and salts thereof comprising preparing 5-(5-fluoro-2-oxo-1,2-dihydro-indol-3Z-ylidenemethyl)-2,4-dimethyl-1H-pyrrole-3-carbonyl substitute of formula 1 according to the process of the present invention, and converting it to sunitinib and salts thereof. Preferably, the sunitinib salt is sunitinib malate.

In another embodiment, the present invention encompasses a process for preparing sunitinib having the following structure: 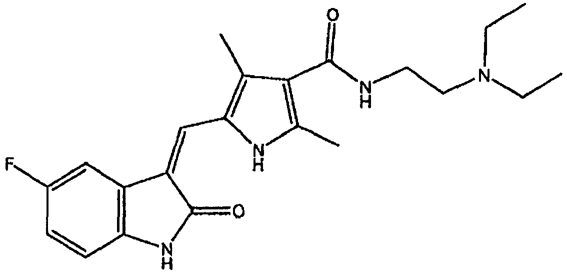 comprising reacting the compound of formula 1: 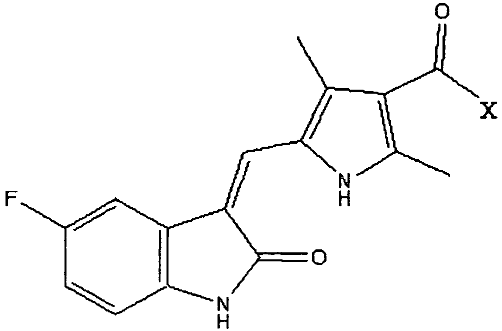 with 2-diethylaminoethylamine of formula 3 of the following structure

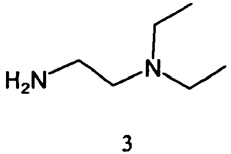

In yet another embodiment, the present invention encompasses a process for preparing sunitinib salts comprising, preparing sunitinib according to the process of the present invention, and converting it to sunitinib salt. Preferably, the sunitinib salt is sunitinib malate.

### BRIEF DESCRIPTION OF THE FIGURES

Figure 1 shows a powder XRD pattern of crystalline Form 1 of 5-(5-fluoro-2-oxo-1, 2-dihydro-indol-(3Z)-ylidenemethyl)-2, 4-dimethyl-1H-pyrrole-3 - carboxylic acid of formula 4.

Figure 2 shows a FTIR spectrum of crystalline Form 1 of 5-(5-fluoro-2-oxo-1, 2-dihydro-indol-(3Z)-ylidenemethyl)-2,4-dimethyl-1H-pyrrole-3-carboxylic acid of formula 4.

Figure 3 shows a powder XRD pattern of crystalline Form 2 of 5-(5-fluoro-2-oxo-1, 2-dihydro-indol-(3Z)-ylidenemethyl)-2, 4-dimethyl-1 H-pyrrole-3-carboxylic acid of formula 4.

Figure 4 shows a FTIR spectrum of crystalline Form 2 of 5-(5-fluoro-2-oxo-1, 2-dihydro-indol-(3Z)-ylidenemethyl)-2,4-dimethyl-1H-pyrrole-3-carboxylic acid of formula 4.

Figure 5 shows a powder XRD pattern of crystalline Form 3 of 5-(5-fluoro-2-oxo-1,2-dihydro-indol-(3Z)-ylidenemethyl)-2,4-dimethyl-1H-pyrrole-3-carboxylic acid of formula 4.

Figure 6 shows a FTIR spectrum of crystalline Form 3 of 5-(5-fluoro-2-oxo-1,2-dihydro-indol-(3Z)-ylidenemethyl)-2,4-dimethyl-1H-pyrrole-3-carboxylic acid of formula 4.

Figure 7 shows a powder XRD pattern of crystalline Form 4 of 5-(5-fluoro-2-oxo-1, 2-dihydro-indol-(3Z)-ylidenemethyl)-2, 4-dimethyl-1H-pyrrole-3-carboxylic acid of formula 4.

Figure 8 shows a FTIR spectrum of crystalline Form 4 of 5-(5-fluoro-2-oxo-1,2-dihydro-indol-(3Z)-ylidenemethyl)-2,4-dimethyl-1H-pyrrole-3-carboxylic acid of formula 4.

Figure 9 shows a PXRD pattern of pyrrolidinium salt of 5-(5-fluoro-2-oxo-1, 2-dihydro-indol-(3Z)-ylidenemethyl)-2, 4-dimethyl-1H-pyrrole-3-carboxylic acid.

### DETAILED DESCRIPTION OF THE INVENTION

The present invention offers processes for the preparation of sunitinib and salts thereof. Preferred embodiments of the invention are capable of achieving higher yields compared to known processes, such as via a new intermediate of the following structure: 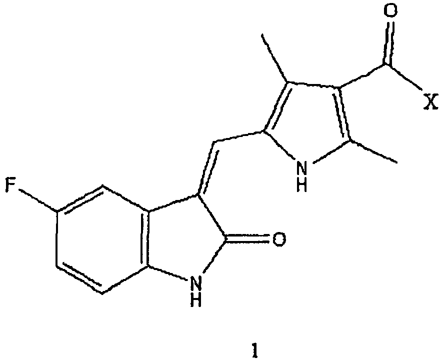 wherein X is either Cl . The preparation of the compound of formula 1, is performed by first conducting a condensation reaction providing the carboxylic acid of formula 4, and then chlorinating it to obtain 5-(5-fluoro-2-oxo-1, 2-dihydro-indol-3Z-ylidenemethyl)-2, 4-dimethyl-1H-pyrrole-3-carbonyl substitute of the following formula 1. Then, the obtained formula 1 is reacted with 2-diethylaminoethylamine of formula 3. Preferably, Sunitinib is produced in a yield of about 80% or greater, preferably at least 82%, and/or purity of at least 99.5% when X is Cl. Preferably, Sunitinib is produced in a yield of about 90% or greater, preferably at least 93%, and/or purity of at least 98% when X is imidazole.

However, when the chlorination is done before the condensation reaction, as described in the following scheme: 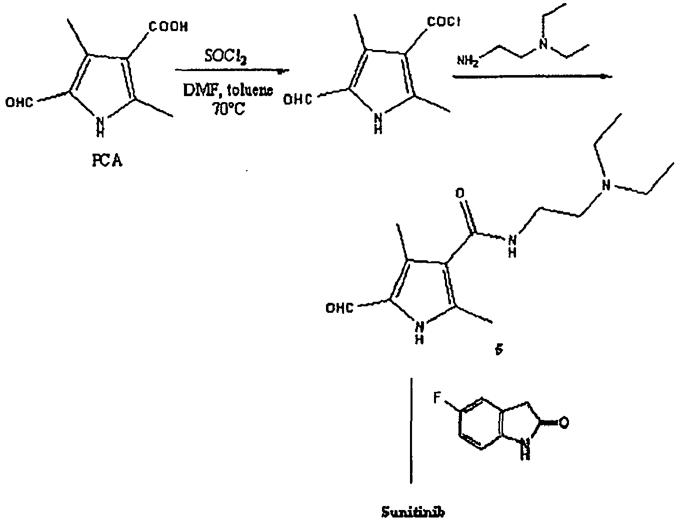 about 48% of the starting PCA remains unreacted. See example 12. In addition, when the process is further continued, by performing the amidation reaction on the mixture containing PCA and its chlorinated derivatives, the compound of formula 5 is formed in a very low yield (3%). See example 12.

When X is Cl, the compound of formula refers to 5-(5-fluoro-2-oxo-1, 2-dihydro-indol-3Z-ylidenemethyl)-2, 4-dimethyl-1H-pyrrole-3-carbonyl chloride, designated formula 1a. 5-(3-fluoro-2-oxo-1,2-dihydro-indol-3Z-ylidenemethyl)-2,4-dimethyl-1H-pyrrole-3-carbonyl chloride can be characterized by data selected from a group consisting of ¹H NMR (DMSO-d6, 400 MHz, 298 K): δ13.84 (s, 1H), 11.03 (s, 1H), 7.78 (dd, J 9.4,2.5 Hz, 1H), 7.69 (s, 1H), 6.90 (ddd, J 9.4,8.5,2.5, 1H), 6.83 (dd, J 8.5,4.6. 1H), 2.51 (s, 3H), 2.48 (s, 3H); ¹³C-NMR (DMSO-d6,100.6 MHz, 298 K): δ 170.0, 166.6, 158.7, 141.3, 135.2, 133.8, 127.4, 126.5, 125.1,116.1, 114.7, 113.1; FTIR: 3168, 3043, 1739, 1676, 1570, 1480,1421,1329, 1195, 1151, 1037, 821, 800; MS: m/z 301, which correspond to (M+H)+ and combination thereof.

The compound of formula 1 can be used to prepare sunitinib and salts thereof having the following structure: 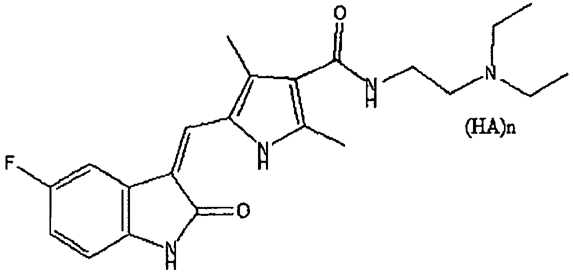 wherein, n is either 0 or 1, HA is a diacid, preferably, malic acid.

When n is 0, the above formula corresponds to sunitinib base ("Sunitinib"). When n is 1, the above formula corresponds to sunitinib salt, preferably, sunitinib malate.

Initially, the process comprises the preparation of formula 1. The process can be illustrated by the following scheme: 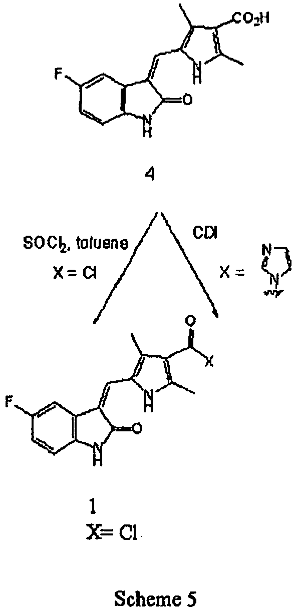 wherein, the carboxylic moiety reacts with chlorinating agent . The process comprises reacting 5-(5-fluoro-2-oxo-1, 2-dihydro-indol-(3Z)-ylidenemethyl)-2,4-dimethyl-1H-pyrrole-3-carboxylic acid of formula 4 with chlorinating agent . Preferably, the chlorinating agent is either thionylchloride or oxalylchloride, more preferably, thionylchloride.

In one embodiment, 5-(5-fluoro-2-oxo-1,2-dihydro-indol-(3Z)-ylidenemethyl)-2,4-dimethyl-1H-pyrrole-3-carboxylic acid of formula 4 is prepared by a process comprising reacting 5-formyl-2,4-dimethyl-1H-pyrrole-3-carboxylic acid (PCA) of the formula: 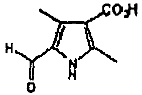 and 5-fluoro-1,3-dihydro-indol-2-one (FDI) of the formula: 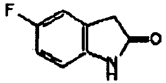 and pyrrolidone, and adjusting the pH to acidic pH at a temperature of about 25°C to about 70°C to obtain a suspension.

Preferably, the reaction comprises combining 5-formyl-2, 4-dimethyl-1H-pyrrole-3-carboxylic acid (PCA), 5-fluoro-1, 3-dihydro-indol-2-one (FDI) and the solvent to obtain a mixture. Preferably, this mixture is combined with pyrrolidine and a second amount of solvent to obtain a suspension.

Preferably, the solvent is selected from a group consisting of ethanol, methanol and mixture thereof.

Preferably, the suspension is stirred for a period of about 5 minutes to about 20 minutes, more preferably, for a period of about 10 minutes to about 1 5 minutes to obtain a solution.

Further, the solution may then be heated to facilitate the reaction. Preferably, heating is done to a temperature of about 40°C to about 70°C more preferably, of about 45°C to about 55°C, most preferably, at about 50°C.

Preferably, heating is done for a period of about 0.5 hours to about 16 hours, more preferably, for a period of about 2 hours to about 6 hours; preferably the pyrrolidinium salt of 5-(5-fluoro-2-oxo-1,2-dihydro-indol-(3Z)-ylidenemethyl)-2, 4-dimethyl-1H-pyrrole-3-carboxylic acid forms and precipitates.

Optionally, the precipitated pyrrolidinium salt of 5-(5-fluoro-2-oxo-1, 2-dihydro-indol-(3Z)-ylidenemethyl)-2, 4-dimethyl-1H-pyrrole-3-carboxylic acid can be recovered.

The recovery of pyrrolidinium salt of 5-(5-fluoro-2-oxo-1, 2-dihydroindol-(3Z)-ylidenemethyl)-2, 4-dimethyl-1H-pyrrole-3-carboxylic acid may be done by cooling and filtering the suspension, washing the precipitate and drying. Preferably, cooling is done to a temperature of about 30°C to about 15°C, more preferably, to a temperature of about 25°C to about 20°C, most preferably, to a temperature of about 25°C. Preferably, the washing is done with methanol.

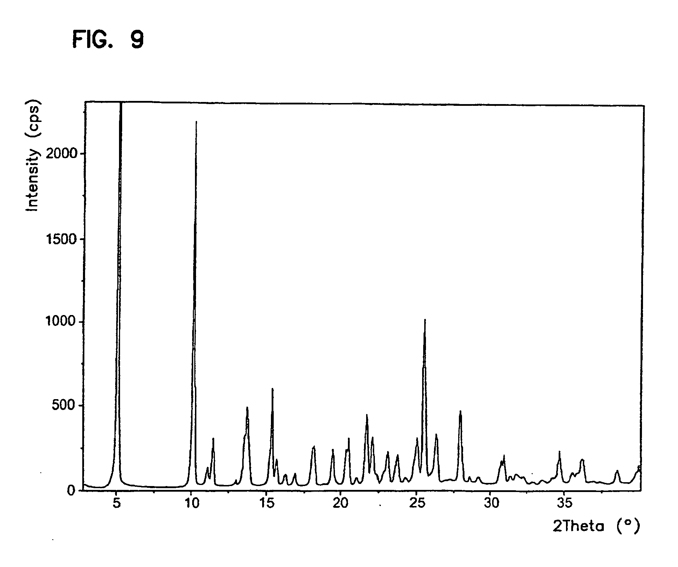

The recovered pyrrolidinium salt of 5-(5-fluoro-2-oxo-1, 2-dihydroindol-(3Z)-ylidenemethyl)-2, 4-dimethyl-1H-pyrrole-3-carboxylic acid may be crystalline. Preferably, it is characterized by a PXRD pattern having peaks at about 5.1, 10.2, 11.5, 13.7, 15.4, 19.5, 21.7, 22.1, 25.5 and 28.0 deg. 2theta ± 0.2 deg and a PXRD pattern as depicted in figure 9.

The recovered pyrrolidinium salt of 5-(5-fluoro-2-oxo-1, 2-dihydroindol-(3Z)-ylidenemethyl)-2, 4-dimethyl-1H-pyrrole-3-carboxylic acid can then be converted to 5-(5-fluoro-2-oxo-1,2-dihydro-indol-(3Z)-ylidenemethyl)-2,4-dimethyl-1H-pyrrole-3-carboxylic acid of formula 4 by adjusting the pH to acidic pH at a temperature of about 25°C to about 70°C, preferably, 40°C to about 60°C to obtain a suspension.

A preferred process comprises suspending the pyrrolidinium salt of 5-(5-fluoro-2-oxo-1, 2-dihydro-indol-(3Z)-ylidenemethyl)-2, 4-dimethyl-1H-pyrrole-3-carboxylic acid in a solvent, preferably water, and heating the suspension to the above temperature prior to adjustment of the pH.

More preferably, the adjustment of the pH is done at a temperature of about 45°C to about 50°C. Most preferably, the adjustment of the pH is done at a temperature of about 50°C.

Typically, the adjustment of the pH is provided by addition of a mineral acid. Preferably, the mineral acid is HCl. The adjustment of the pH provides an acidic pH, preferably, the pH is to about 0 to about 5.0, more preferably, to about 1.0 to about 3.0.

Preferably, the adjustment of the pH at the above temperature provides a suspension from which 5-(5-fluoro-2-oxo-1, 2-dihydro-indol-(3Z)-ylidenemethyl)-2, 4-dimethyl-1H-pyrrole-3-carboxylic acid of formula 4 is recovered easily due to enhanced filterability.

The recovered 5-(5-fluoro-2-oxo-1, 2-dihydro-indol-(3Z)-ylidenemethyl)-2, 4-dimethyl-1H-pyrrole-3-carboxylic acid of formula 4 can be washed and dried. The washing is done with a solvent and water. Preferably, the washing in the recovery step is done first with the solvent and then with water. Preferably, the solvent in the recovery step is either ethanol or methanol. Preferably, the drying is done at a temperature of about 60°C to about 80°C. Preferably, the drying is conducted for a period of about 16 hours.

In a preferred embodiment, the obtained 5-(5-fluoro-2-oxo-1, 2-dihydro-indol-(3Z)-ylidenemethyl)-2, 4-dimethyl-1H-pyrrole-3-carboxylic acid of formula 4 is crystalline. Reported herein are four crystalline forms of 5-(5-fluoro-2-oxo-1, 2-dihydro-indol-(3Z)-ylidenemethyl)-2, 4-dimethyl-1H-pyrrole-3-carboxylic acid of formula 4.

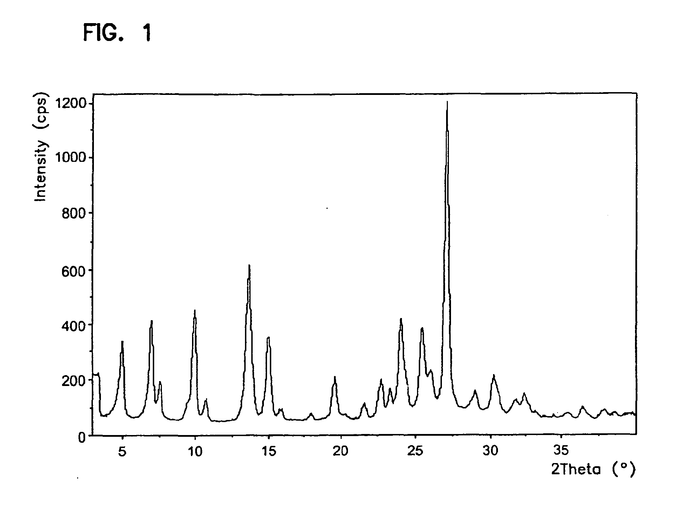

The first crystalline form of 5-(5-fluoro-2-oxo-1,2-dihydro-indol-(3Z)-ylidenemethyl)-2,4-dimethyl-1H-pyrrole-3-carboxylic acid of formula 4 is characterized by data selected from a group consisting of PXRD pattern having peaks at about 5.0, 7.0, 7.6, 10.0, 10.7, 13.7, 15.0, 19.6, 22.7, 24.1, 25.5, 27.1 and 30.2 deg. 2theta ± 0.2 deg. 2theta and PXRD pattern as depicted in Figure 1.

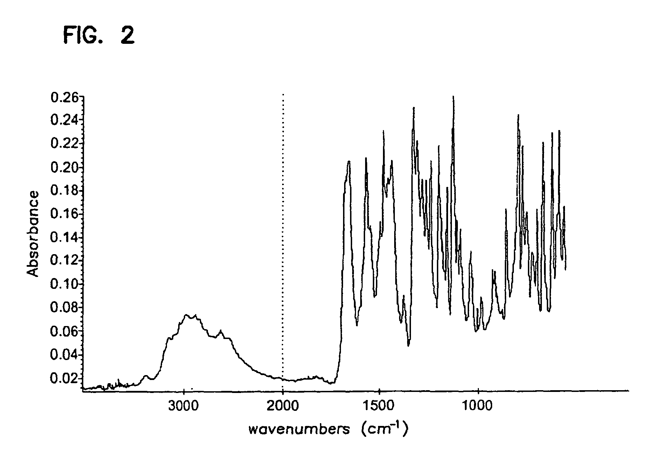

The first crystalline form of 5-(5-fluoro-2-oxo-1,2-dihydro-indol-(3Z)-ylidenemethyl)-2,4-dimethyl-1H-pyrrole-3-carboxylic acid of formula 4 may be further characterized by FTIR spectrum as depicted in Figure 2 .

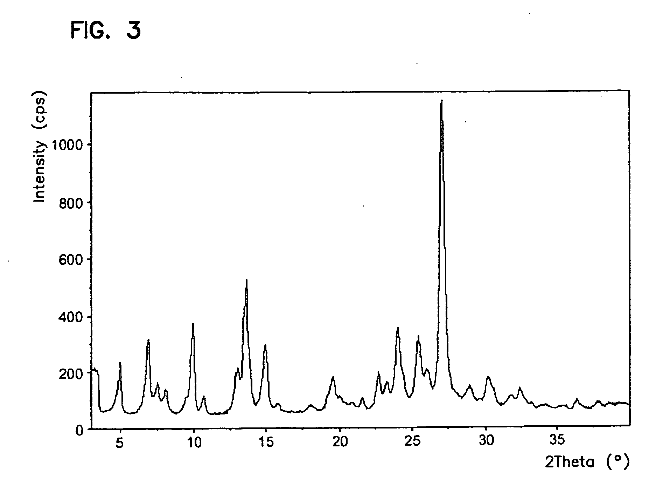

The second crystalline form of 5-(5-fluoro-2-oxo-1,2-dihydro-indol-(3Z)-ylidenemethyl)-2,4-dimethyl-1H-pyrrole-3-carboxylic acid of formula 4 is characterized by data selected from a group consisting of PXRD pattern having peaks at about 5.0, 6.9, 7.5, 8.1, 9.9, 13.6, 14.9, 19.5 and 27.1 deg. 2theta ± 0.2 deg. 2theta and PXRD pattern as depicted in Figure 3.

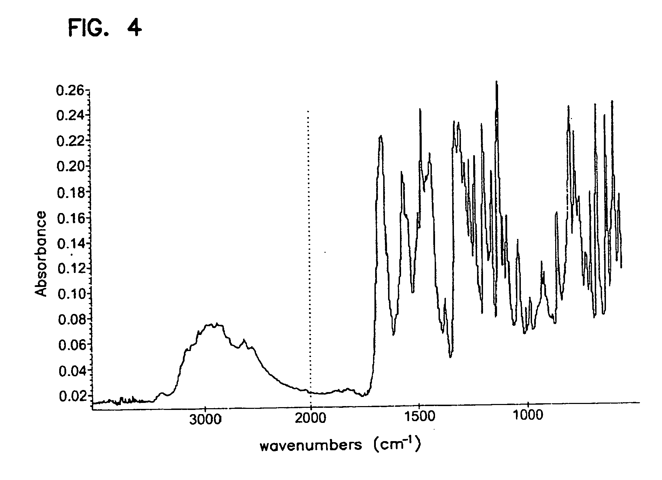

The second crystalline form of 5-(5-fluoro-2-oxo-1,2-dihydro-indol-(3Z)-ylidenemethyl)-2,4-dimethyl-1H-pyrrole-3-carboxylic acid of formula 4 may be further characterized by FTIR spectrum as depicted in Figure 4.

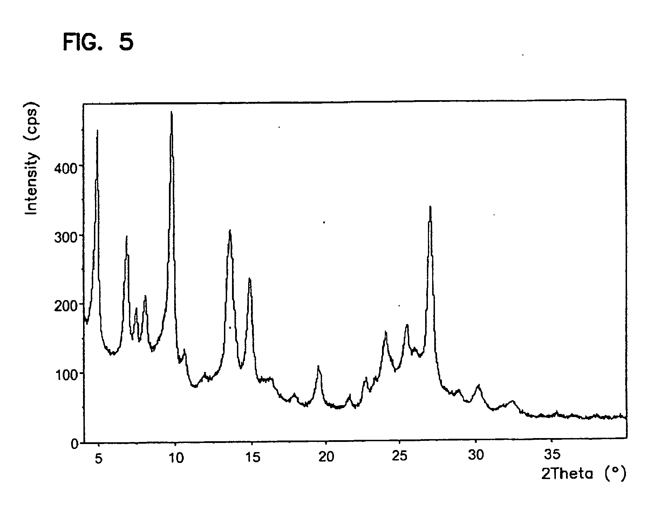

The third crystalline form of 5-(5-fluoro-2-oxo-1,2-dihydro-indol-(3Z)-ylidenemethyl)-2,4-dimethyl-1H-pyrrole-3-carboxylic acid of formula 4 is characterized by data selected from a group consisting of PXRD pattern having peaks at about 4.8, 6.9, 7.4, 9.8, 10.6, 13.6, 14.8 and 27.1 deg. 2theta±0.2 deg. 2theta and PXRD pattern as depicted in Figure 5.

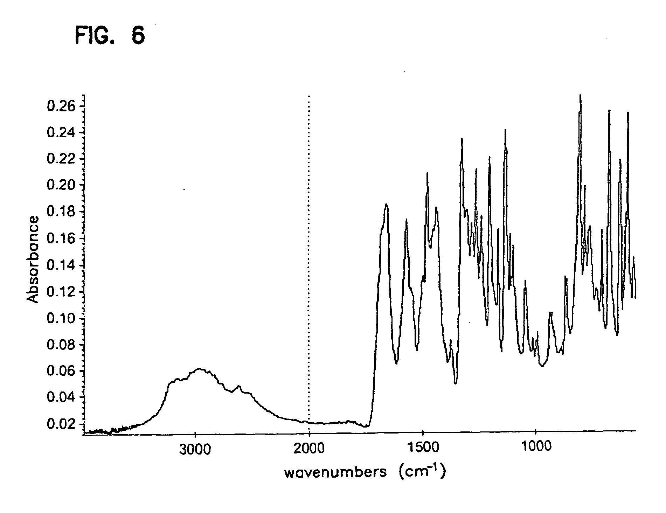

The third crystalline form of 5-(5-fluoro-2-oxo-1,2-dihydro-indol-(3Z)-ylidenemethyl)-2,4-dimethyl-1H-pyrrole-3-carboxylic acid of formula 4 may be further characterized by FTIR spectrum as depicted in Figure 6.

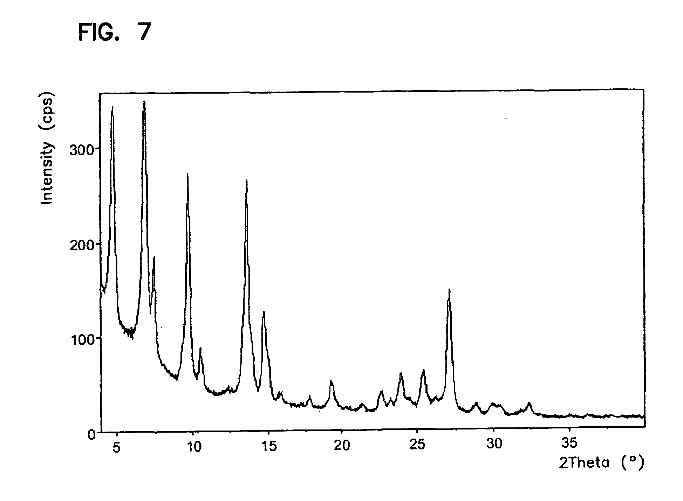

The forth crystalline form of 5-(5-fluoro-2-oxo-1,2-dihydro-indol-(3Z)-ylidenemethyl)-2,4-dimethyl-1H-pynole-3-carboxylic acid of formula 4 is characterized by data selected from a group consisting of PXRD pattern having peaks at about 5.0, 7.0, 7.6, 8.1, 9.9, 13.0, 13.7, 14.9, 20.0, 24.1, 25.5, 27.1 and 30.2 deg. 2theta ± 0.2 deg. 2theta and PXRD pattern as depicted in Figure 7.

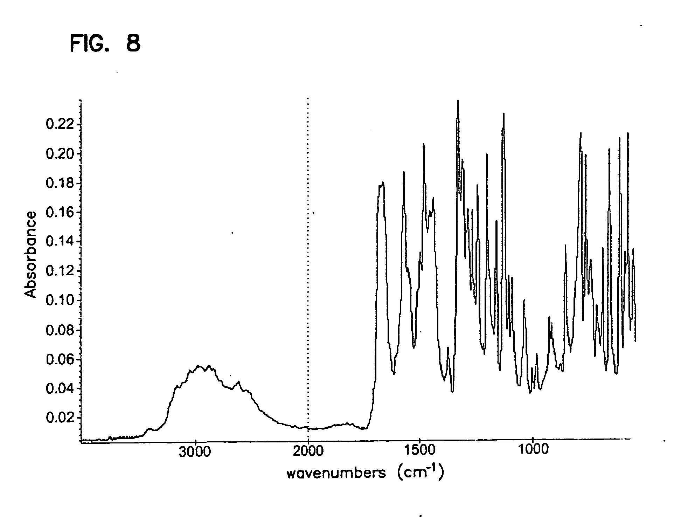

The forth crystalline form of 5-(5-fluoro-2-oxo-1,2-dihydro-indol-(3Z)-ylidenemethyl)-2,4-dimethyl-1H-pyrrole-3-carboxylic acid of formula 4 may be further characterized by FTIR spectrum as depicted in Figure 8.

The above described crystalline forms of 5-(5-fluoro-2-oxo-1, 2-dihydro-indol-(3Z)ylidenemethyl)-2, 4-dimethyl-1H-pyrrole-3-carboxylic acid of formula 4, can be used to prepare 5-(5-fluoro-2-oxo-1,2-dihydro-indol-3Z-ylidenemethyl)-2,4-dimethyl-1H-pyrrole-3-carbonyl substitute of formula 1.

As described before the process comprises reacting 5-(5-fluoro-2-oxo-1, 2-dihydro-indol-(3Z)-ylidenemethyl)-2, 4-dimethyl-1H-pyrrole-3-carboxylic acid of formula 4 either with chlorinating agent

When X is Cl, the compound of formula 1 refers to 5-(5-fluoro-2-oxo-1, 2-dihydro-indol-3Z-ylidenemethyl)-2, 4-dimethyl-1H-pyrrole-3-carbonyl chloride, designated formula 1a.

When X is imidazole, the compound of formula 1 refers to 5-(5-fluoro-2-oxo-1, 2-dihydro-indol-3Z-ylidenemethyl)-2, 4-dimethyl-1H-pyrrole-3-(carbonyl-1-imidazole), designated formula 1b.

When 5-(5-fluoro-2-oxo-1,2-dihydro-indol-3Z-ylidenemethyl)-2,4-dimethyl-1H-pyrrole-3-carbonyl chloride, designated formula 1a, is prepared, a preferred process comprises reacting 5-(5-fluoro-2-oxo-1,2-dihydro-indol-(3Z)-ylidenemethyl)-2,4-dimethyl-1H-pyrrole-3-carboxylic acid of formula 4 with thionyl chloride in the presence or absence of a catalyst. Preferably, the catalyst is DMF.

Preferably, the mole ratio between 5-(5-fluoro-2-oxo-1,2-dihydroindol-(3Z)-ylidenemethyl)-2,4-dimethyl-1H-pyrrole-3-carboxylic acid of formula 4 and thionyl chloride is of about 1:1.3 to about 1:1.8 respectively, more preferably, of about 1:1.4.

Preferably, the mole ratio between 5-(5-fluoro-2-oxo-1,2-dihydroindol-(3Z)-ylidenemethyl)-2,4-dimethyl-1H-pyrrole-3-carboxylic acid of formula 4 and DMF is of about 1:0.1 to about 1:0.3, more preferably, of about 1: 0.2.

Typically, the reaction is done in the presence of a solvent. Preferably, the reaction with thionyl chloride is done in the presence of a solvent selected from a group consisting of: an aromatic hydrocarbon, cyclic ether and mixtures thereof.

Preferably, the aromatic hydrocarbon is C₆-C₉ aromatic hydrocarbon, more preferably, is selected from the group consisting of chlorobenzene, and toluene, most preferably, toluene. Preferably, the cyclic ether is C₄-C5 cyclic ether, more preferably, is either tetrahydrofuran or methyl-tetrahydrofuran.

Typically, the above reaction is maintained for a sufficient time at a given temperature to allow the formation of the compound of formula 1. Preferably, the reaction is maintained with stirring. Preferably, the reaction is maintained at a temperature of about room temperature to about reflux. Preferably, the reaction with thionyl chloride is done at temperature of about 40°C to about 80°C, more preferably, at a temperature of about 65°C to about 75°C, most preferably, of about 70°C.

The above reaction is preferably maintained for a period of about 4 hours to about overnight. Preferably, the reaction with thionyl chloride is maintained for a period of about 3 hours to about 5 hours, more preferably, for a period of about 4 hours.

The above reactions result in a suspension comprising 5-(5-fluoro-2-oxo-1, 2-dihydro-indol-3Z-ylidenemethyl)-2, 4-dimethyl-1H-pyrrole-3-carbonyl substitute of formula 1.

The precipitated 5-(5-fluoro-2-oxo-1,2-dihydro-indol-3Z-ylidenemethyl)-2,4-dimethyl-1H-pyrrole-3-carbonyl substitute of formula 1 can then be recovered. The recovery may be done, for example, by cooling the heated suspension, filtering it, washing and drying under vacuum. Preferably, drying is done at a temperature of about 50°C to about 60°C, preferably, for about 10 hours to about 18 hours.

Preferably, in the reaction with thionyl chloride the recovery process includes cooling to about room temperature. Preferably, the cooling is done for a period of about 1 hour to about 3 hours, more preferably for a period of about 2 hours.

The obtained 5-(5-fluoro-2-oxo-1,2-dihydro-indol-3Z-ylidenemethyl)-2, 4-dimethyl-1 H-pyrrole-3-carbonyl substitute of formula 1 is preferably recovered in high yield. For example, when X is Cl, the obtained 5-(5-fluoro-2-oxo-1, 2-dihydro-indol-3Z-ylidenemethyl)-2,4-dimethyl-1H-pyrrole-3-carbonyl chloride of formula 1a a is preferably recovered in yield of at least 97.8%.

5-(5-fluoro-2-oxo-1,2-dihydro-indol-3Z-ylidenemethyl)-2,4-dimethyl-1H-pyrrole-3-carbonyl substitute of formula I can be converted to sunitinib and salts thereof, as shown below.

In one embodiment, the conversion to sunitinib having the following structure 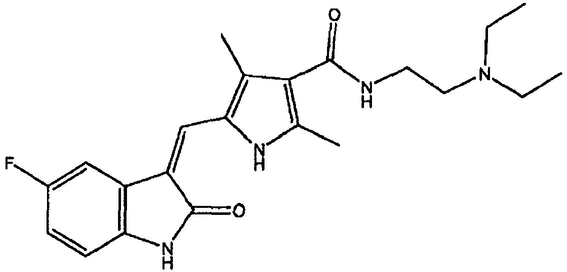 comprises reacting the compound of formula 1 having the following formula: 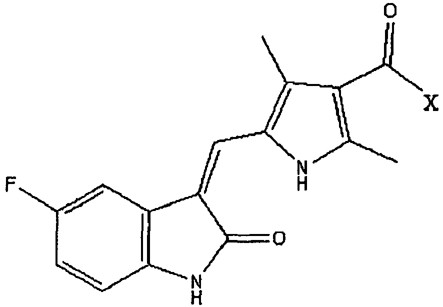 with 2-diethylaminoethylamine of formula 3 having the following structure: 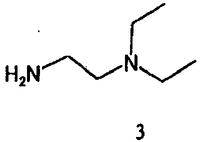 wherein X is either Cl. Typically, this reaction occurs in the presence of a solvent.

When X is Cl the reaction is preferably done in the presence of a solvent selected from the group consisting of toluene, 2-methyl tetrahydrofuran, tetrahydrofuran, dimethylformamide and 1-methyl-2-pyrrolidone. More preferably, in the presence of 2-methyl tetrahydrofuran as a solvent.

Typically, excess of thionyl chloride can be removed by distillation, prior to the reaction with diethylenediamine of formula 3.

Preferably, the distillation is done at a temperature of about 40°C to about 60°C, more preferably at about 50°C. Preferably, distillation is done under vacuum.

Typically, the reaction is maintained, preferably under stirring to allow the formation of sunitinib. Preferably, the reaction is maintained for a period of about 1 hour to about 24 hours, more preferably, for about 1 hour to about 5 hours. Preferably, the reactions are maintained at a temperature of about room temperature to about 70°C.

Preferably, when X is Cl the reaction is done for a period of about 0.5 to about 3 hours. More preferably, for a period of about 1 hour. Preferably, the reaction is done at a temperature of about 25°C to about 80°C, more preferably at about 40°C.

The obtained sunitinib can then be recovered. The recovery process of sunitinib may comprise adding water to the reaction mixture to precipitate Sunitinib, filtering off the precipitated sunitinib, washing and drying.

Preferably, when X is Cl the recovery further comprises concentrating the obtained suspension, prior to the filtration, providing a new suspension.

Preferably, the concentration is done by evaporating some of the solvent at a temperature of about 40°C to about 60°C, more preferably 50°C. Preferably, the evaporation is done under vacuum.

To increase the yield, the obtained new suspension is stirred, preferably, for a period of about 1 hour to about 3 hours, more preferably for about 2 hours.

Preferably, drying is done at a temperature of about 50°C to about 80°C, more preferably at about 50°C to about 60°C. Preferably, drying is done for period of about 4 hours to about overnight, more preferably, for about 10 hours to about 16 hours.

Preferably, when X is Cl the drying is done at a temperature of about 70°C to about 80°C, more preferably at about 80°C. Preferably, the drying is done for a period of about 10 hours to about 16 hours.

Typically, the recovered sunitinib can then be converted to sunitinib salt, preferably, to sunitinib malate. The conversion can be done by reacting sunitinib base with an acid, preferably, malic acid. When the acid is malic acid, the conversion can be done, for example, according to the process disclosed in U.S. publication No. 2003/0069298, hereby incorporated by reference.

Optionally, sunitinib can be purified prior to the conversion to sunitinib salt. Preferably, the purification comprises acidifying sunitinib to obtain sunitinib salt, and then converting it back to sunitinib by reacting the salt with a base.

The process comprises dissolving Sunitinib in a mixture of water with an acid to obtain sunitinib salt. Preferably, the acid is an inorganic acid, more preferably, hydrochloric acid. Then, said solution is extracted either with ketone, preferably, methyl-isobutyl ketone or with 2-Methyl THF, providing a two-phase system. Typically, the phases are separated and a base is added to the aqueous phase providing sunitinib. Preferably, when the reaction is performed in 2-Methyl THF, the extraction is done with 2-Methyl THF.

Preferably, the base is aqueous ammonia. Preferably, the aqueous phase is basified to a pH of about 8 to about 9, more preferably, to a pH of about 8.5, to obtain a suspension comprising a precipitation of sunitinib in forms of crystals.

The crystalline sunitinib can then be recovered. The recovery process may comprise filtering off the precipitated sunitinib, washing and drying. Preferably, drying is done at a temperature of about 70°C to about 80°C. Preferably, drying is done for a period of about 10 hours to about 16 hours.

### EXAMPLES

PXRD

XRD diffraction was performed on X-Ray powder diffractometer: PanAlytical X'pert Pro powder diffractometer, CuKα radiation, λ = 1.541874 Å. X'Celerator detector active length (2 theta) = 2.122 mm, laboratory temperature 22-25°C, zero background sample-holders. Prior to analysis the samples were gently ground by means of mortar and pestle in order to obtain a fine powder. The ground sample was adjusted into a cavity of the sample holder and the surface of the sample was smoothed by means of a cover glass slide.

### FTIR

FTIR spectra were collected by means of a spectrometer Nicolet Nexus. ATR technique was used for the measurement with the following settings:

| | |
|---|---|
| Range: | 4000 - 550 cm⁻¹ |
| Number of sample scans: | 64 |
| Resolution: | 4.000 |
| Apodization: | Happ-Genzel |
| Sample gain: | 8.0 |
| Final format: | Absorbance |

The empty ATR crystal was measured as a background under the same conditions as were the samples. The resulting record was then subtracted automatically from the spectra of the samples.

### Example 1: Preparation of sunitinib via 5-(5-fluoro-2-oxo-1, 2-dihydro-indol-3Z-ylidenemethyl)-2, 4-dimethyl-1H-pyrrole-3-carbonyl chloride

31.2 g of 5-(5-fluoro-2-oxo-1, 2-dihydro-indol-3Z-ylidenemethyl)-2,4-dimethyl-1H-pyrrole-3-carboxylic acid, obtained as described in US 7125905, were refluxed under stirring for 4 hours in one liter flask with 310 g of toluene, 15 g of thionyl chloride and 1 g of dimethylformamide.

The stirred suspension was cooled at room temperature for 2 hours and filtered; the cake was washed with 50 g of toluene and dried at 50° under vacuum overnight.

Yield was 32. 4 g (97.8%) of a compound corresponding by NMR and MS to the expected structure.

20 g of diethylendiamine were dissolved in one liter flask with 300 g of tetrahydrofuran; about 200 g of solvent were distilled away at 50° under vacuum.

20 g of 5-(5-fluoro-2-oxo-1,2-dihydro-indol-3Z-ylidenemethyl)-2,4-dimethyl-1H-pyrrole-3-carbonyl chloride, prepared as above, were added under stirring and solution obtained was left for one hour to react without more heating. 300 g of water were added and suspension was evaporated at 50° under vacuum to eliminate most of organic solvent. After stirring 2 hours at room temperature the suspension was filtered, washed with 100 g of water and dried at 50° under vacuum overnight, obtaining 23.5 g of crude Sunitinib.

### Purification

Crude material was dissolved with 560 g of water and 190 g of 1 M Hydrochloric acid, extracted with 200 g of methyl-isobutyl ketone.

Clarified aqueous phase was basified under stirring with concentrated aqueous ammonia to pH 8.5 and after 2 hours the suspension was filtered and crystals were washed with 100 g of water.

Product was dried at 50° under vacuum overnight obtaining 20.5 (82% yield, 99.6% purity by HPLC) of sunitinib.

### Example 2: Preparation of Sunitinib via 5-(5-fluoro-2-oxo-1, 2-dihydro-indol-3Z-ylidenemethyl)-2, 4-dimethyl-1H-pyrrole-3-(carbonyl-1-imidazole)

4.6 g of 5-(5-fluoro-2-oxo-1, 2-dihydro-indol-3Z-ylidenemethyl)-2,4-dimethyl-1H-pyrrole-3-carboxylic acid, obtained as described in US7125905, were stirred for 4 hours in 0.1 liter flask with 46 g of 1-methyl-2-pyrrolidone and 3 g of 1,1'-carbonyldiimidazole (CDI), after this time 0.7 g of CDI were added and reaction was left stirring overnight.

46 g of water were added under stirring and after 1 hour the suspension was filtered and the cake washed with water.

Product was dried at 60° under vacuum obtaining 5.1 g (95% yield); NMR and MS confirmed the expected structure.

1 g of 5-(5-fluoro-2-oxo-1,2-dihydro-indol-3Z-ylidenemethyl)-2,4-dimethyl-1H-pyrrole-3-(carbonyl-1-imidazole), prepared as above, was added to 10 g of 1-methyl-2-pyrrolidone and 0.5 g of diethylendiamine under stirring and the mixture was left for one day to react at 70°.

10 g of water were added and after 2 hour at room temperature the suspension was filtered, the cake was washed with water and was dried at 60° under vacuum for 4 hours to constant weight.

1.06 g of crude product (93% yield, 98% purity by HPLC) was obtained.

### Example 3: Conversion of Sunitinib to Sunitinib Malate (according to Example 1, Preparation A of U.S. publication No. 2003/0069298)

### Preparation of the Anhydrous Crystal Form I of the L-Malice Acid Salt of N-[2-(Diethylamino) ethyl]-5-[(5fluoro-1, 2-dihydro-2-oxo-3H-indol- 3-ylidene) methyl]-2, 4-dimethyl-1H-pyrrole-3-carboxamide.

### Preparation A:

N-[2-(Diethylamino)ethyl]-5-[(5-fluoro-1,2-dihydro-2- -oxo-3H-indol-3-ylidene)methyl]-2,4-dimethyl-1H-pyrrole-3-carboxamide (130 mg, 0.326 mMol) was added to 20 mL methanol, and the mixture was stirred. L-malic acid (47.2 mg, 0.352 mMol) was added, resulting in rapid dissolution of all the solids. The methanol was removed under reduced pressure to produce a poorly crystalline orange solid. Acetonitrile (5 mL) was added, and the slurry was stirred and heated for about 10 minutes. Stirring was continued while the slurry was allowed to cool to room temperature. The crystals were filtered and dried, resulting in 149 mg of solids (86% yield).

### Example 4- preparation of crystalline form 1 of 5-(5-fluoro-2-oxo-1, 2-dihydro-indol-(3Z)-ylidenemethyl)-2, 4-dimethyl-1H-pyrrole-3-carboxylic acid of formula 4

In a reactor under nitrogen atmosphere, 450 g of PCA (1.0 eq), 447.6 g of FDI (1.1 eq) and 9 L of absolute ethanol were loaded and vigorously stirred at room temperature. Then 229.95 g of pyrolidine (1.2 eq) with 447 mL of ethanol were added and the suspension was stirred 10-15 minutes to dissolution.

The mixture was then heated to 50 °C and stirred at this temperature for 8 hours (precipitation of the product occurs during the heating). Then the mixture was neutralized with 1860 g of hydrochloric acid 2 mol.L⁻¹ and the suspension was kept at 50 °C for 2 hours.

After this step, the mixture was cooled to room temperature for 2 hours and then the solid was filtered on gooch P3 and washed with 2.7 L of ethanol. The filtered product was washed with 13.5 L of water. It was dried at 80°C overnight under vacuum yielding 777 g of 5-(5-fluoro-2-oxo-1,2-dihydro-indol-(3Z)-ylidenemethyl)-2,4-dimethyl-1H-pymrrole-3-carboxylic acid derivative with 96.1% total yield.

### Example 5- preparation of crystalline form 2 of 5-(5-fluoro-2-oxo-1, 2-dihydro-indol-(3Z)-ylidenemethyl)-2, 4-dimethyl-1H-pyrrole-3-carboxylic acid of formula 4

In a reactor under nitrogen atmosphere, 277 g of PCA (1.0 eq), 275.5 g of FDI (1.1 eq) and 5.54 L of absolute ethanol were loaded and vigorously stirred at room temperature. Then 141.54 g of pyrrolidine (1.2 eq) with 275 mL of ethanol were added and the suspension was stirred 10-15 minutes to dissolution.

Then the mixture was heated to 50 °C and stirred at this temperature for 8 hours (precipitation of the product occurs during the heating).

The mixture was neutralized with 1144 g of hydrochloric acid 2 mol.L⁻¹ and the suspension was kept at 50 °C for 2 hours.

After this step, the mixture was cooled to room temperature for 2 hours and then the solid was filtered on gooch P3 and washed with 1.66 L of ethanol. The filtered product was washed with 8.3 L of water. It was dried at 80 °C overnight under vacuum yielding 448 g of 5-(5-fluoro-2-oxo-1,2-dihydro-indol-(3Z)-ylidenemethyl)-2,4-dimethyl-1H-pyrrole-3-carboxylic acid derivative with 90.0% total yield.

### Example 6- preparation of crystalline form 3 of 5-(5-fluoro-2-oxo-1, 2-dihydro-indol-L3Z)-ylidenemethyl)-2, 4-dimethyl-1H-pyrrole-3-carboxylic acid of formula 4

In a reactor under nitrogen atmosphere, 23 g of PCA (1.0 eq). 26.3 g of FDI (1.265 eq) and 633 mL of absolute ethanol were loaded and vigorously stirred at room temperature. Then 26 g of pyrrolidine (3 eq) were added and the suspension was stirred 10-15 minutes to dissolution.

The mixture was then heated to reflux and stirred at this temperature for 6 hours (precipitation of the product occurs during the heating).

Then the mixture was cooled to room temperature and the solid was filtered on gooch P3 and washed with 100 mL of ethanol. The obtained product was loaded again into the reactor and it was suspended into 200 mL of a mixture acetone/water 40/60 and 17.3 g of HCl 37% were added. The suspension was stirred for 2 hours at 25 °C and then filtered on gooch P3 washing the solid with 200 mL of water. It was dried at 60 °C for a night under vacuum yielding 32.6 g of 5-(5-fluoro-2-oxo-1,2-dihydro-indol-(3Z)-ylidenemethyl)-2,4-dimethyl-1H-pyrole-3-carboxylic acid derivative.

### Example 7- preparation of crystalline form 4 of 5-(5-fluoro-2-oxo-1, 2-dihydro-indol-(3Z)-ylidenemethyl)-2,4-dimethyl-1H-pyrrole-3-carboxylic acid of formula 4

In a reactor under nitrogen atmosphere, 20 g of PCA (1.0 eq). 19.9 g of FDI (1.1 eq) and 400 mL of absolute ethanol were loaded and vigorously stirred at room temperature. Then 11.9 mL of pyrrolidine (1.2 eq) were added and the suspension was stirred 10-15 minutes to dissolution.

The mixture was then heated to 50 °C and stirred at this temperature for 6hours (precipitation of the product occurs during the heating).

Then the temperature was maintained at 50 °C and 68 mL of HCl 2 mol.L⁻¹ were slowly added up to pH 1.5 - 3.0. The suspension was stirred for 2 hours at 50 °C and then filtered on gooch P3 washing the solid with 2x50 mL of ethanol. It was dried at 60 °C for a night under vacuum, loaded again into the filter and washed with 3x150 mL of water.

The orange solid was dried in oven under vacuum at 60 °C for 16 hours yielding 27 g of 5-(5-fluoro-2-oxo-1,2-dihydro-indol-(3Z)-ylidenemethyl)-2,4-dimethyl-1H-pyrrole-3-carboxylic acid derivative.

### Example 8 preparation of 5-(5-fluoro-2-oxo-1, 2-dihydro-indol-(3Z)-ylidenemethyl)-2, 4-dimethyl-1H-pyrrole-3-carboxylic acid of formula 4 in methanol

In a reactor under nitrogen atmosphere 5g of 5-formyl-2, 4-dimethyl-1H-pyrrole-3-carboxylic acid (PCA) (1.0eq), 4.97g of 5-fluoro-1, 3-dihydro-indol-2-one (FDI) (1.1eq) and 75ml of methanol were loaded and vigorously stirred at room temperature. Then 2.97ml of pyrrolidine (1.2eq) were added and the suspension was stirred 10-15 minutes to dissolution.

The mixture was then heated to 50°C and stirred at this temperature for 2-3 hours (precipitation of the product occurs during the heating).

Then, maintaining the temperature at 50°C, 20ml of HCl 2M were slowly added up to pH 1.5-3.0. The suspension was stirred for 1 hour at 50°C and then filtered on gooch P3 washing the solid with 2x12.5ml of methanol and with 3x50ml of water.

The obtained product was dried at 60°C for a night under vacuum yielding 8.4g of Sunitinib carboxylic acid derivative.

### Example 9- Preparation of Sunitinib via Sunitinib carboxylic acid derivative

In a 500 ml reactor, 15.0 g of Sunitinib carboxylic acid derivative (Compound 4) were suspended into 300ml of toluene (ratio 20/1.0 v/w. starting material) under vigorous stirring at room temperature. 0.755 g. of dimethylformamide (ratio 0.2/1.0 w/w) was added to the mixture.

The temperature was set at 70°C and at this temperature, 5.1 g. of thionyl chloride (ratio 1.4/1.0 w/w) were dropped in a range of sixty minutes. The reaction was kept at 70°C for 7 hours under stirring.

Then 140 ml of solvent were distilled to remove excess of thionyl chloride from the suspension and the reaction filtered on gooch P3 washing with 3v/w of toluene. The wet solid (sunitinib acyl chloride derivative) was re-loaded into the reactor and 300ml Methyl-tetrahydrofuran loaded and stirred. Then the reaction mixture was heated to 70°C and 6.35g of 2-diethylamino-ethylamine (ratio 1.1/1.0 w/w starting material) were dropped in five minutes at 70°C. After one hour the reaction was completed and 150 ml of water and HCl 2N until pH 2 were added to the suspension.

The mixture was filtered using a decalite pad to obtain a clarified phase. The two phases were separated at 50°C and the organic phase discarded. The aqueous phase was washed once more with 300ml of Methyl-tetrahydrofuran at 50°C under stirring. The two phases separated again and the organic phase discarded. The aqueous phase was then basified to pH 8.5 with 5% ammonia solution at 50 °C. After one hour stirring, the suspension was filtered on gooch P3 and the wet solid dried at 60°C under vacuum overnight.

15.9 g. of sunitinib base were obtained with a purity of NLT 99.5% by HPLC.

### Example 10- preparation of 5-(5-fluoro-2-oxo-1, 2-dihydro-indol-(3Z)-ylidenemethyl)-2, 4-dimethyl-1H-pyrrole-3-carboxylic acid

In a reactor under nitrogen atmosphere 10g of 5-formyl-2, 4-dimethyl-1H-pyrrole-3-carboxylic acid (PCA) (1.0eq), 9.94g of 5-fluoro-1, 3-dihydro-indol-2-one (FDI) (1.1eq) and 150ml of methanol were loaded and vigorously stirred at room temperature. Then 5.94ml of pyrrolidine (1.2eq) was added and the suspension was stirred 10-15 minutes to dissolution. The mixture was then heated to 50°C and stirred at this temperature for 2-3 hours (precipitation of the product occurred during the heating).

The pyrrolidinium salt of 5-(5-fluoro-2-oxo-1, 2-dihydro-indol-(3Z)-ylidenemethyl)-2, 4-dimethyl-1H-pyrrole-3-carboxylic acid thus obtained was cooled to 25°C, filtered on gooch P3 and washed with 50ml of methanol. The wet solid (24g) was then loaded again into the reactor and suspended into 150ml of water and the mixture heated to 50°C.

Then, maintaining the temperature at 50°C, 23ml of HCl 2M was slowly added up to pH 1.5-3.0. The suspension was stirred for 1 hour at 50°C and then filtered on gooch P3 washing the solid with 2x50ml of water.

The obtained product was dried at 75°C for a night under vacuum yielding 15.5g of 5-(5-fluoro-2-oxo-1, 2-dihydro-indol-(3Z)-ylidenemethyl)-2, 4-dimethyl-1H-pyrrole-3-carboxylic acid.

### Example 11- Preparation of Sunitinib via Sunitinib carboxylic acid derivative

In a 500 ml reactor, 15.0 g. of Sunitinib carboxylic acid derivative (Compound 4) was suspended into 300ml of toluene (ratio 20/1.0 v/w. starting material) under vigorous stirring at room temperature. 0.755 g. of dimethylformamide (ratio 0.2/1.0 mol of SM) were added to the mixture.

The temperature was set at 70°C and at this temperature, 5.1 g. of thionyl chloride (ratio 1.4/1.0 mol of SM) were dropped in a range of sixty minutes. The reaction was kept at 70°C for 7 hours under stirring.

Then 140 ml of solvent were distilled to remove excess of thionyl chloride from the suspension and the reaction was filtered on gooch P3 was washed with 3v/w of toluene. The wet solid (sunitinib acyl chloride derivative) was re-loaded into the reactor and 225ml Methyl-tetrahydrofuran were loaded under stirring. Then the reaction mixture was heated to 40°C and 6.35g of 2-diethylamino-ethylamine (ratio 1.1/1.0 w/w starting material) were dropped in five minutes at 40°C. After one hour the reaction was completed and 225 ml of water and HCl 2N until pH 2 were added to the suspension.

The mixture was filtered using a decalite pad to obtain a clarified phase. The two phases were separated at 40°C and the organic phase was discarded. The aqueous phase was washed once more with 225ml of Methyl-tetrahydrofuran at 40°C under stirring. The two phases were separated again and the organic phase was discarded.

The aqueous phase was then basified to pH 8.5 with 5% ammonia solution at 40°C. After one hour stirring, the suspension was filtered on gooch P3 and the wet solid was dried at 80°C under vacuum overnight.

16.5 g. of sunitinib base were obtained (83% yield) with a purity of NLT 99.5% by HPLC.

### Comparative Example 12- unsuccessful chlorination of pyrrole carboxylic acid with thionylchloride

In a 100 ml reactor, 5.0 g. of PCA were suspended into 75ml of toluene under vigorous stirring at room temperature. 15 ml of toluene are thus distilled at 50°C under vacuum reaching a final volume of 50ml (10 volumes on weight SM).

At 50°C, 0.44 g. of dimethylformamide (ratio 0.2/1.0 mol of SM) and 5g of thionyl chloride (ratio 1.4/1.0 mol of SM) were added to the mixture.

The reaction was kept at 50°C for 3 hours under stirring. The HPLC control reveals still 48% unreacted pyrrole and no changing with respect to the control done after 2 hours. The reaction looks very dark with a presence of a lot of tars.

Then 15ml of solvent were distilled to remove excess of thionyl chloride from the suspension and then other 15ml are added to reach the starting 75ml of toluene.

Maintaining at 50°C, 3.83g of N, N'-diethylaminoethylamine (ratio 1.1/1.0 w/w starting material) were dropped in five minutes. After one hour the reaction was completed and 50 ml of water and HCl 2N until pH 2 were added to the suspension.

The precipitate was filtered and the two phases separated, the aqueous phase was basified with NaOH 2M to pH 9.0 and extracted with 70ml of dichloromethane. Difficult separation is observed, the extraction is done with a volume of 200ml of water and 500ml of dichloromethane.

The aqueous phase once more extracted with another 500ml of dichloromethane. The organic phase is then evaporated to residue and triturated with a mixture hexane/ethylether 3:1.

The obtained solid is filtered on gooch P3 and dried in oven under vacuum at 35°C, 0.25g of the desired product are obtained (3%yield, 80% purity).

### Example 13 - chlorination

In a 100 ml reactor, 6.0g of Sunitinib Carboxylic acid derivative were suspended into 60ml of toluene under vigorous stirring at room temperature. 30 ml of toluene are thus distilled at 50°C under vacuum reaching a final volume of 60ml (10 volumes on weight SM).

At 70°C, 1.24 ml of dimethylformamide (ratio 0.8/1.0 mol of SM) and 9.72ml of thionyl chloride (ratio 6.5/1.0 mol of SM) were added to the mixture. The reaction was kept at 70°C for 8 hours under stirring then it is cooled to room temperature and filtered on gooch P3, washed with 20ml of toluene and the obtained solid used as is.

3g of the solid is suspended in 20ml of Me-THF and, at 50°C, 1.45ml of N, N'-diethylaminoethylamine (ratio 1.1/1.0 w/w starting material) were dropped in five minutes. After one hour the reaction was completed. Sunitinib was obtained.

### Example 14 - chlorination

In a 100 ml reactor, 6.0g of Sunitinib Carboxylic acid derivative were suspended into 60ml of toluene under vigorous stirring at room temperature. 30 ml of toluene are thus distilled at 50°C under vacuum reaching a final volume of 60ml (10 volumes on weight SM).

At 40°C, 0.31 ml of dimethylformamide (ratio 0.2/1.0 mol on SM) and 1.75ml of thionyl chloride (ratio 1.2/1.0 mol on SM) were added to the mixture. The reaction was kept at 40°C for 7 hours and it is checked by HPLC. Formula 1 (when X is Cl) was obtained.

## Claims

1. A compound of the following formula 1: 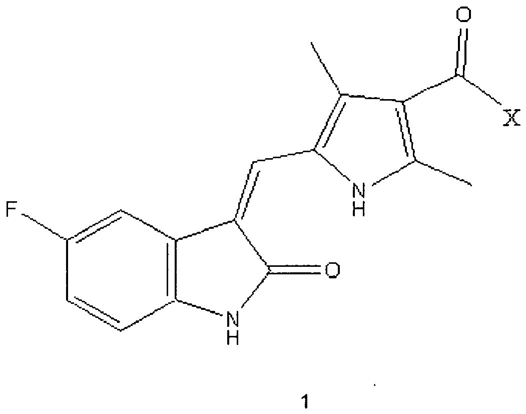 wherein X is Cl.

2. The use of the compound of claim 1 for the preparation of sunitinib or a salt thereof having the following structure: 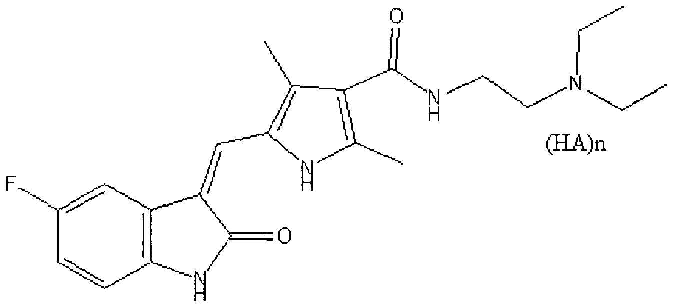 wherein n is 0 or 1 and HA is a diacid, preferably wherein n is 1 and HA is malic acid.

3. A process for the preparation of the compound of claim 1, comprising reacting 5-(5-fluoro-2-oxo-1,2-dihydro-indol-(3Z)-ylidenemethyl)-2, 4-dimethyl-1H-pyrrole-3-carboxylic acid (compound 4) 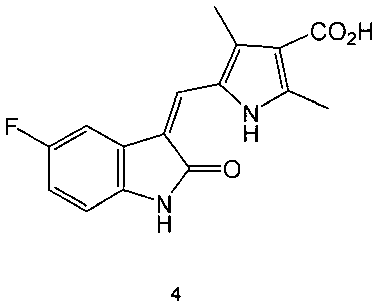 with a chlorinating agent, and further comprising the step of recovering the compound of formula 1.

4. The process of claim 3, wherein compound 4 reacts with a chlorinating agent selected from the group consisting of thionyl chloride and oxalyl chloride.

5. The process of any of claims 3 and 4, further comprising DMF.

6. The process of any of claims 3 to 5, wherein the mole ratio between 5-(5-fluoro-2-oxo-1,2-dihydro-indol-(3Z)-ylidenemethyl)-2,4-dimethyl-1H-pyrrole-3-carboxylic acid of formula 4 and thionyl chloride is about 1:1.3 to about 1:1.8 respectively.

7. The process of any of claims 3 to 6, wherein mole ratio between 5-(5-fluoro-2-oxo-1,2-dihydro-indol-(3Z)-ylidenemethyl)-2,4-dimethyl-1H-pyrrole-3-carboxylic acid of formula 4 and DMF is of about 1:0.1 to about 1:0.3.

8. The process of any of claims 3 to 7, wherein compound 4 reacts with thionyl chloride in the presence of a solvent selected from a group consisting of an aromatic hydrocarbon and a cyclic ether,
preferably wherein the solvent is a C₆-C₉ aromatic hydrocarbon,
most preferably wherein the solvent is selected from the group consisting of chlorobenzene and toluene.

9. The process of claim 8, wherein the solvent is a C₄-C₅ cyclic ether,
preferably wherein the cyclic ether is either tetrahydrofuran or methyl-tetrahydrofuran.

10. The process of any of claims 3 to 9, wherein the reaction with thionyl chloride is done at temperature of about 40°C to about 80°C.

11. The process of any of claims 3 to 10, wherein the compound of formula 4 is prepared by a process comprising reacting 5-formyl-2,4-dimethyl-1H-pyrrole-3-carboxylic acid (PCA) of the formula: 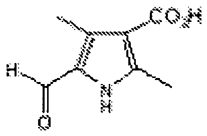 and 5-fluoro-1,3-dihydro-indol-2-one (FDI) of the formula: 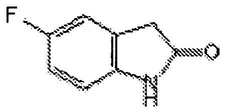 in the presence of pyrrolidine, and adjusting the pH to acidic pH at a temperature of about 25°C to about 70°C to obtain a suspension containing compound 4.

12. A process for preparing sunitinib or a salt thereof having the following structure: 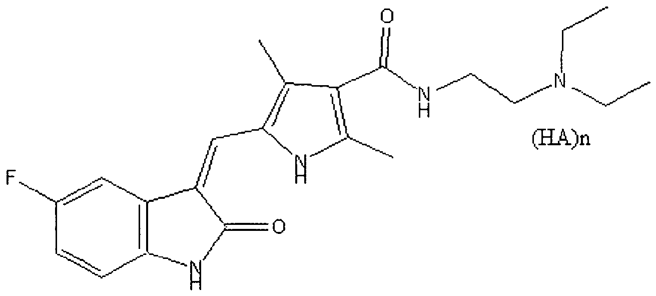 wherein n is either 0 or 1 and HA is a diacid, comprising
preparing the compound of formula 1 according to any of claims 3 to 11, and converting it to sunitinib or a salt thereof,
preferably wherein the converting step comprises reacting the compound of formula 1 with 2-diethylaminoethylamine.

13. The process of claim 12, wherein X is Cl in the compound of formula 1 and the reaction occurs in the presence of a solvent selected from the group consisting of toluene, 2-methyl tetrahydrofuran, tetrahydrofuran and 1-methyl-2-pyrrolidone, preferably wherein the solvent is 2-methyl tetrahydrofuran.

14. The process of claim 13, further comprising the step of recovering sunitinib or a salt thereof.

## Patentansprüche

1. Verbindung der folgenden Formel 1: 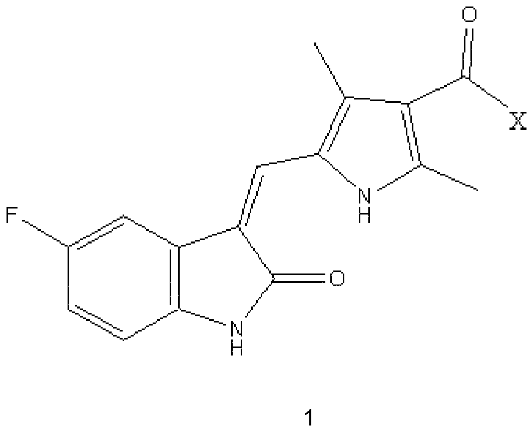 wobei X Cl ist.

2. Verwendung der Verbindung der Formel nach Anspruch 1 zur Herstellung von Sunitinib oder von einem Salz davon mit der folgenden Struktur: 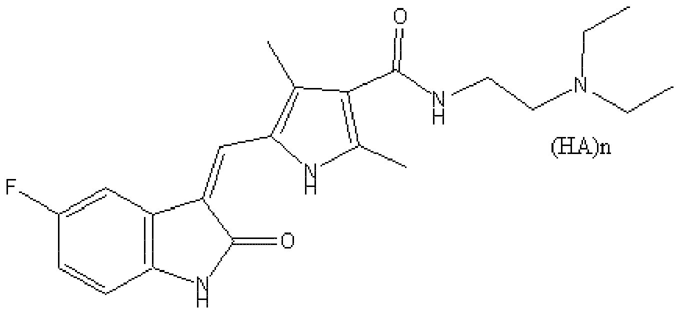 wobei n 0 oder 1 ist und HA eine Disäure ist, vorzugsweise wobei n 1 ist und HA Äpfelsäure ist.

3. Verfahren zur Herstellung der Verbindung nach Anspruch 1, umfassend die Umsetzung von 5-(5-Fluor-2-oxo-1,2-dihydro-indol-(3Z)-ylidenmethyl)-2,4-dimethyl-1 H-pyrrol-3-carboxylsäure (Verbindung 4) 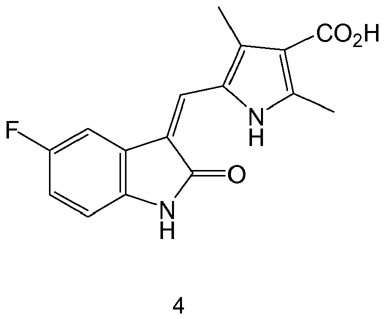 mit einem Chlorierungsmittel, und weiterhin umfassend den Schritt des Gewinnens der Verbindung der Formel 1.

4. Verfahren nach Anspruch 3, wobei die Verbindung 4 mit einem Chlorierungsmittel reagiert, das aus der Gruppe ausgewählt ist bestehend aus Thionylchlorid und Oxalylchlorid.

5. Verfahren nach einem der Ansprüche 3 und 4, weiterhin umfassend DMF.

6. Verfahren nach einem der Ansprüche 3 bis 5, wobei das Molverhältnis zwischen 5-(5-Fluor-2-oxo-1,2-dihydro-indol-(3Z)-ylidenmethyl)-2,4-dimethyl-1H-pyrrol-3-carboxylsäure der Formel 4 und Thionylchlorid jeweils etwa 1:1,3 bis etwa 1:1,8 beträgt.

7. Verfahren nach einem der Ansprüche 3 bis 6, wobei das Molverhältnis zwischen 5-(5-Fluor-2-oxo-1,2-dihydro-indol-(3Z)-ylidenmethyl)-2,4-dimethyl-1 H-pyrrol-3-carboxylsäure der Formel 4 und DMF von etwa 1:0,1 bis etwa 1:0,3 beträgt.

8. Verfahren nach einem der Ansprüche 3 bis 7, wobei die Verbindung 4 mit Thionylchlorid in Gegenwart von einem Lösungsmittel reagiert, das aus der Gruppe ausgewählt ist, bestehend aus einem aromatischen Kohlenwasserstoff und einem cyclischen Ether,
vorzugsweise wobei das Lösungsmittel ein C₆-C₉-aromatischer Kohlenwasserstoff ist, am stärksten bevorzugt wobei das Lösungsmittel aus der Gruppe ausgewählt ist bestehend aus Chlorbenzol und Toluol.

9. Verfahren nach Anspruch 8, wobei das Lösungsmittel ein C₄-C₅-cyclischer Ether ist, vorzugsweise wobei der cyclische Ether entweder Tetrahydrofuran oder Methyltetrahydrofuran ist.

10. Verfahren nach einem der Ansprüche 3 bis 9, wobei die Reaktion mit Thionylchlorid bei einer Temperatur von etwa 40°C bis etwa 80°C durchgeführt wird.

11. Verfahren nach einem der Ansprüche 3 bis 10, wobei die Verbindung der Formel 4 durch ein Verfahren hergestellt wird, umfassend die Umsetzung von 5-Formyl-2,4-dimethyl-1H-pyrrol-3-carboxysäure (PCA) der Formel: 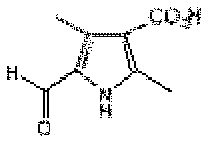 und 5-Fluor-1,3-dihydro-indol-2-on (FDI) der Formel: 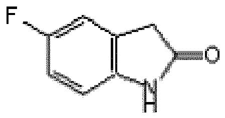 in Gegenwart von Pyrrolidin, und Einstellen des pH auf sauren pH bei einer Temperatur von etwa 25°C bis etwa 70°C um eine Suspension zu erhalten, die die Verbindung 4 enthält.

12. Verfahren zur Herstellung von Sunitinib oder einem Salz davon mit der folgenden Struktur: 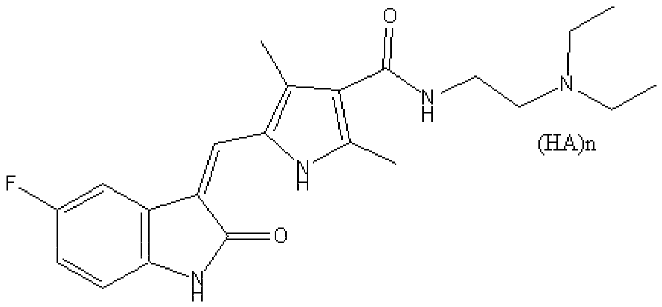 wobei n entweder 0 oder 1 ist und HA eine Disäure ist, umfassend Herstellen der Verbindung der Formel 1 nach einem der Ansprüche 3 bis 11, und deren Umwandeln in Sunitinib oder ein Salz davon,
vorzugsweise wobei der Umwandlungschritt das Umsetzen der Verbindung der Formel 1 mit 2-Diethylaminoethylamin umfasst.

13. Verfahren nach Anspruch 12, wobei X in der Verbindung der Formel 1 Cl ist und die Umsetzung in Gegenwart von einem Lösungsmittel erfolgt, das aus der Gruppe ausgewählt ist, bestehend aus Toluol, 2-Methyltetrahydrofuran, Tetrahydrofuran und 1-Methyl-2-pyrrolidon, vorzugsweise wobei das Lösungsmittel 2-Methyltetrahydrofuran ist.

14. Verfahren nach Anspruch 13, weiterhin umfassend den Schritt bestehend aus dem Gewinnen von Sunitinib oder von einem Salz davon.

## Revendications

1. Composé de la formule 1 : 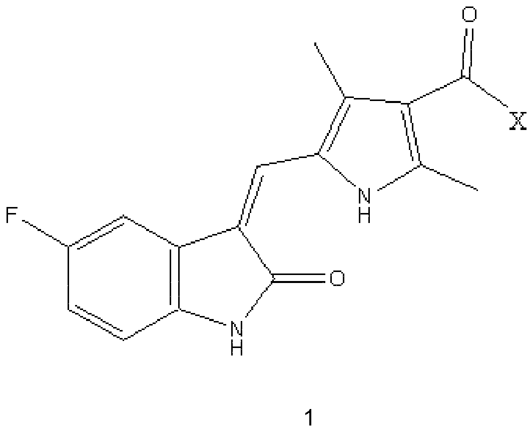 dans lequel X est CI.

2. Utilisation du composé selon la revendication 1 pour la préparation du sunitinib ou d'un sel de celui-ci ayant la structure suivante : 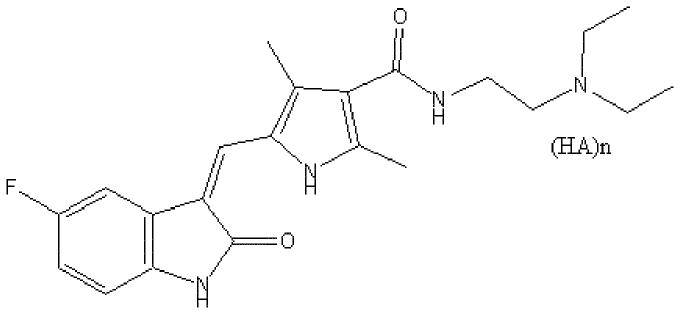 dans laquelle n est 0 ou 1 et HA est un diacide, préférablement dans laquelle n est 1 et HA est l'acide malique.

3. Procédé pour la préparation du composé selon la revendication 1, comprenant l'étape consistant à faire réagir l'acide 3-carboxylique de 5-(5-fluoro-2-oxo-1,2-dihydro-indol-(3Z)-ylidèneméthyl)-2,4-diméthyl-1 H-pyrrole (composé 4) 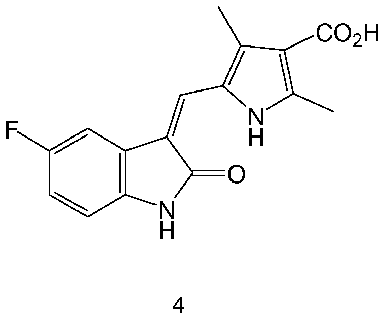 avec un agent de chloruration, et comprenant en outre l'étape consistant à récupérer le composé de la formule 1.

4. Procédé selon la revendication 3 dans lequel le composé 4 réagit avec un agent de chloruration sélectionné parmi le groupe consistant en le chlorure de thionyle et le chlorure d'oxalyle.

5. Procédé selon l'une des revendications 3 et 4, comprenant en outre du DMF.

6. Procédé selon l'une des revendications 3 à 5, dans lequel le rapport molaire entre l'acide 3-carboxylique de 5-(5-fluoro-2-oxo-1,2-dihydro-indol-(3Z)-ylidèneméthyl)-2,4-diméthyl-1 H-pyrrole de la formule 4 et le chlorure de thionyle est environ 1:1,3 à environ 1:1,8, respectivement.

7. Procédé selon l'une des revendications 3 à 6, dans lequel le rapport molaire entre l'acide 3-carboxylique de 5-(5-fluoro-2-oxo-1,2-dihydro-indol-(3Z)-ylidèneméthyl)-2,4-diméthyl-1 H-pyrrole de la formule 4 et le DMF est environ 1:0,1 à environ 1:0,3.

8. Procédé selon l'une des revendications 3 à 7, dans lequel le composé 4 réagit avec le chlorure de thionyle en présence d'un solvant sélectionné parmi le groupe consistant en un hydrocarbure aromatique et un éther cyclique,
préférablement dans lequel le solvant est un hydrocarbure aromatique en C₆-C₉, encore plus préférablement dans lequel le solvant est sélectionné parmi le groupe consistant en le chlorobenzène et le toluène.

9. Procédé selon la revendication 8, dans lequel le solvant est un éther cyclique en C₄-C₅, préférablement dans lequel l'éther cyclique est le tétrahydrofurane ou bien le méthyl-tétrahydrofurane.

10. Procédé selon l'une des revendications 3 à 9, dans lequel la réaction avec le chlorure de thionyle est faite à une température d'environ 40°C à environ 80°C.

11. Procédé selon l'une des revendications 3 à 10, dans lequel le composé 4 est préparé selon un procédé comprenant l'étape consistant à faire réagir l'acide 3-carboxylique de 5-formyl-2,4-diméthyl-1 H-pyrrole (PCA) de la formule : 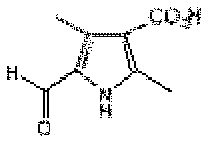 et le 5-fluoro-1,3-dihydro-indol-2-one (FDI) de la formule : 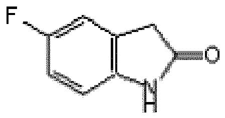 en présence de la pyrrolidine, et à ajuster le pH à un pH acidique à une température d'environ 25°C à environ 70°C pour obtenir une suspension contenant le composé 4.

12. Procédé pour la préparation du sunitinib ou d'un sel de celui-ci ayant la structure suivante : 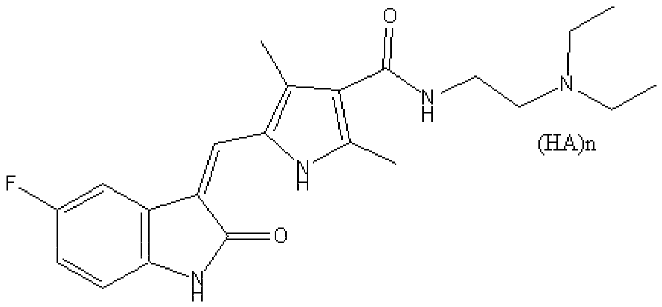 dans lequel n est 0 ou bien 1 et HA est un diacide, comprenant l'étape consistant à préparer le composé de la formule 1 selon l'une des revendications 3 à 11, et le convertir en sunitinib ou en un sel de celui-ci,
préférablement dans lequel l'étape de conversion comprend la réaction du composé de la formule 1 avec la 2-diéthylaminoéthlyamine.

13. Procédé selon la revendication 12, dans lequel X est CI dans le composé de la formule 1 et la réaction se produit en présence d'un solvant sélectionné parmi le groupe consistant en le toluène, le 2-méthyl tétrahydrofurane, le tétrahydrofurane, et le 1-méthyl-2-pyrrolidone, préférablement dans lequel le solvant est le 2-méthyl tétrahydrofurane.

14. Procédé selon la revendication 13, comprenant en outre l'étape consistant à récupérer le sunitinib ou un sel de celui-ci.
